# FASCICULE DE BREVET EUROPEEN

(11) **EP 0 409 740 B1**
(45) Date de publication et mention de la délivrance du brevet: **03.11.1993**
(21) Numéro de dépôt: 90402100.3
(22) Date de dépôt: 20.07.1990
(51) Int. Cl.: C07C 65/24, C07B 59/00, A61K 49/00, G01N 33/534

(54) **Nouveau composé marqué au tritium, sa préparation et son application, notamment dans le repérage des récepteurs nucléaires des rétinoides**
Mit Tritium markierte Verbindung, seine Herstellung und Verwendung, besonders bei der Lokalisierung von nuklearen Rezeptoren von Retinoiden
Compound labelled with tritium, its preparation and use, especially in locating retinoid nuclear receptors

(30) Priorité: 20.07.1989 FR 8909778
(43) Date de publication de la demande: 23.01.1991
(73) Titulaire: CENTRE INTERNATIONAL DE RECHERCHES DERMATOLOGIQUES GALDERMA, ( CIRD GALDERMA), 06560 Valbonne (FR)
(72) Inventeur: Shroot, Braham Villa 35, F-06600 Antibes (FR); Darmon, Michel, F-06600 Antibes (FR)
(74) Mandataire: Tonnellier, Jean-Claude

(56) Documents cités:
- EP-A- 0 199 636
- DE-A- 3 142 975
- GB-A- 2 194 535

## Description

La présente invention a pour objet un nouveau composé marqué au tritium apparenté aux rétinoïdes, sa préparation et son application notamment dans la détermination de l'affinité des rétinoïdes pour leurs récepteurs nucléaires et/ou dans la détermination de la teneur cellulaire en récepteurs nucléaires. La particularité et l'avantage majeur de ce composé est son absence d'affinité pour la protéine de liaison cytosolique (CRABP).

On sait que les rétinoïdes constituent une classe connue de composés qui agissent notamment sur la prolifération et la différenciation de nombreux types de cellules; voir par exemple B.A. Pawson et coll., Journal of Medicinal Chemistry, vol. 25, n°11, pages 1269-1277 (1982).

Les rétinoïdes ont été utilisés en particulier dans le traitement de diverses maladies dermatologiques dans lesquelles un dérèglement des mécanismes de contrôle de la prolifération et de la différenciation des cellules épidermiques est impliqué; voir par exemple l'ouvrage "Update : Dermatology in General Medicine, edited by Thomas B.Fitzpatrick et al. (MacGraw-Hill Book Company), publié en 1983, en particulier le chapitre de D.B.Windhorst et al., The Retinoids, pages 226-237.

Le mode d'action des rétinoïdes est analogue à celui des stéroïdes et d'autres effecteurs interagissant avec des récepteurs nucléaires ; Chytil & Ong, Proc. Fed. Am. Soc. Exp.Biol. vol 38, 2510-2514 (1979). Des récepteurs nucléaires de l'acide rétinoïque (RARs) ont été isolés ; Daly & Redfern, Eur.J.Biochem., vol 168, 133-139 (1987) et les ADN complémentaires correspondants clonés et séquencés ; Petkovich et al., Nature, vol 330, 444-450 (1987); Brand et al., Nature, vol. 332, 850-853 (1988). Jusqu'à présent, deux récepteurs ont été décrits chez l'homme, RARα(462 résidus) et RAR β (448 résidus). Il a d'autre part été montré qu'il existe dans le cytosol une protéine de liaison appelée CRABP (cellular retinoic acid binding protein), mais son rôle est mal connu.

La détermination de l'affinité d'un rétinoïde pour les RARs constitue un moyen particulièrement intéressant d'évaluation biologique potentielle dudit rétinoïde. Cependant, la présence de CRABP peut gêner cette détermination d'affinité pour les RARs dans le cas des rétinoïdes classiques.

Plusieurs méthodes expérimentales permettent de déterminer l'affinité d'un ligand pour ses récepteurs ou protéines de liaison. On peut en particulier opérer par une méthode directe si le ligand considéré est marqué radioactivement, ou encore par compétition avec un ligand radioactif si le ligand considéré n'est pas marqué radioactivement.

Un bon ligand radioactif doit d'une part présenter une affinité élevée pour ses récepteurs et, d'autre part, présenter une faible fixation sur toute autre molécule. En outre, il doit être suffisamment stable pour permettre une utilisation commode.

Dans le cas des rétinoïdes acides, le ligand radioactif généralement utilisé est le plus souvent l'acide rétinoïque tritié, soit en position 2 (J.Labelled Compounds and Radiopharmaceuticals XVIII, p.1099 (1980)), soit en position 4 (demande de brevet allemand n°3.142.945).

L'acide rétinoïque présente certes une excellente affinité pour les RARs, avec une fixation non spécifique faible, mais la molécule présente l'inconvénient de se dégrader rapidement, par radiolyse, oxydation et photolyse. D'autre part, l'acide rétinoïque est aussi capable de se fixer sur la CRABP.

On a maintenant découvert que l'acide 6-[3-(1-adamantyl)-4- méthoxyphényl]-2-naphtoïque tritié constitue un nouveau ligand radioactif qui présente une excellente affinité pour les RARs, mais aucune affinité pour la CRABP. De plus, ce nouveau ligand est très stable et peut être obtenu avec une haute activité spécifique, ce qui le rend particulièrement utile pour la mesure de l'affinité des rétinoïdes pour les RARS et particulièrement dans des conditions où la CRABP est aussi présente.

La présente invention a donc pour objet l'acide 6-[3-(1-adamantyl)-4-méthoxyphényl]-2-naphtoïque marqué au tritium, et notamment en position 5 du groupement adamantyl méthoxyphényl et en position 5 de la partie naphtoïque.

L'invention a notamment pour objet un nouveau ligand radioactif, tel que défini ci-dessus, ayant une activité spécifique supérieure à 10 Ci/mmole (environ 370 GBq/mmole) et de préférence au moins égale à 30 Ci/mmole, soit environ 1110 GBq/mmole.

L'invention a également pour objet un procédé de préparation du nouveau ligand radioactif tel que défini ci-dessus. Ce procédé est principalement caractérisé par le fait que l'on prépare un alkyl ester multihalogéné d'acide 6-[3-(1-adamantyl)-4-méthoxyphényl] -2-naphtoïque et qu'on le soumet à l'action d'un agent réducteur tritié puis à l'action d'un agent de saponification.

L'halogène est de préférence le brome.

L'agent réducteur est notamment le tritium. La réaction de réduction est effectuée selon les méthodes connues, par exemple en présence d'un catalyseur comme le palladium, à température ambiante.

L'alkyl ester tritié obtenu après la réduction est ensuite saponifié selon les méthodes usuelles, par exemple par une base alcaline telle que la soude, en milieu méthanolique.

Le dérivé halogéné utilisé comme produit de départ est notamment un dérivé dihalogéné, de préférence le dérivé bromé. Le dérivé dihalogéné est en particulier le dérivé halogéné en position 5 du groupement adamantylméthoxyphényl et en position 5 de la partie naphtoïque. L'alkyl ester utilisé est de préférence un ester d'alkyle inférieur (1 à 6 C) tel que l'ester méthylique.

L'alkyl ester multi halogéné de l'acide 6-[3-(1-adamantyl)-4-méthoxyphényl]-2-naphtoïque peut lui-même être préparé à partir de l'alkyl ester de l'acide 6-[3-(1-adamantyl)-4-méthoxyphényl]-2-naphtoïque par l'action d'un agent d'halogénation (en particulier de bromation). Par exemple, le dérivé bromé peut être obtenu par l'action du brome dans le dichlorométhane à température ambiante et sous atmosphère d'azote.

L'acide 6-[3-(1-adamantyl)-4-méthoxyphényl]-2-naphtoïque est un produit connu (appelé ci-après composé I), de même que ses esters. Ils peuvent être obtenus selon le procédé décrit dans la demande de brevet européen n°86.400785 (199.636).

L'invention a également pour objet, à titre de produit intermédiaire obtenu dans le procédé décrit ci-dessous, l'acide 6-[3-(1-adamantyl)-4 méthoxy-5-halogénophényl]-5-halogéno-2-naphtoïque, et en particulier le dérivé dibromé.

L'invention a aussi pour objet l'utilisation de l'acide 6-[3-(1-adamantyl)-4-méthoxyphényl]-2-naphtoïque tritié comme marqueur radioactif notamment dans la détermination de l'affinité, de la présence ou de la teneur des rétinoïdes et/ou dans la quantification des RARs.

Par exemple, le marqueur radioactif de l'invention peut être utilisé lors de la purification des RARs par les méthodes classiques de chromatographie d'exclusion sous basse ou haute pression : il suffit d'ajouter aux cellules une quantité déterminée du ligand radioactif. Les RARs se trouvent ainsi marqués et on peut facilement repérer leur présence ou leur absence dans une fraction donnée.

La connaissance des courbes de fixation sur les RARs permet également l'utilisation du marqueur de l'invention dans le repérage, la quantification ou le marquage des RARs endogènes ou obtenus par des méthodes de transfection, en particulier dans les cas où de la CRABP contaminante est présente.

L'invention a également pour objet l'utilisation de l'acide 6-[3-(1-adamantyl)-4-méthoxyphényl]-2-naphtoïque tritié comme marqueur radioactif, notamment pour mesurer l'affinité de substances telles que des rétinoïdes pour les RARs, selon une méthode de compétition ;

Le marqueur radioactif de l'invention peut également être utilisé :
- dans la caractérisation d'anticorps contre le composé I, ces anticorps (obtenus selon les méthodes classiques d'obtention d'anticorps anti-haptènes) étant eux-mêmes utilisables dans la détermination de la quantité du composé I fixé ou non sur les RARs ; on peut utiliser conjointement le composé I et lesdits anticorps pour réaliser un radioimmunodosage ;
- dans l'étude de la distribution in vivo ainsi que cellulaire et subcellulaire du composé I, sans interférence avec la CRABP.

Le marqueur radioactif de l'invention peut aussi être utilisé dans l'étude du mécanisme d'action intracellulaire et du métabolisme général du composé I in vivo et dans les cellules et extraits cellulaires.

Ces utilisations sont mises en oeuvre selon les méthodes usuelles.

L'exemple suivant illustre l'invention sans toutefois la limiter.

### EXEMPLE 1

Acide 6-[3-(1-adamantyl)-4-méthoxyphényl]-2-naphtoïque marqué au tritium :
a) Un mélange de l'ester méthylique correspondant à l'acide cité (1 g ; 2,4 mmoles) et de 4,5 ml de brome dans 30 ml de dichlorométhane est agité pendant 24 heures.
   Le mélange est ajouté à 50 ml d'eau, puis extrait au dichloro- méthane (150ml).
   La phase organique est séparée, lavée à l'eau puis à l'aide d'une solution saturée d'hydrogénocarbonate de sodium et enfin de thiosulfate de sodium.
   On sèche sur sulfate de magnésium anhydre, puis le solvant est évaporé sous pression réduite.
   On obtient 1,24 g d'un solide jaune orangé correspondant à un produit dibromé d'après son spectre de masse. La position des atomes de brome est confirmée par analyse du spectre RMN du proton, en comparaison avec le spectre RMN du produit non bromé. Le produit obtenu est l'ester méthylique de l'acide 6-[3-(1-adamantyl)-4methoxy-5 bromophényl]-5-bromo-2-naphtoïque.
b) Le composé bromé obtenu est ensuite tritié de la façon suivante:
   On dissout 10mg du composé dibromé dans 2ml d'éthanol et 1ml de THF.
   On ajoute 17µl de triéthylamine et 14mg de palladium sur charbon à 10%.
   On agite pendant 2 heures sous atmosphère de tritium, puis on élimine le catalyseur par filtration.
   On élimine ensuite le solvant par évaporation sous pression réduite. Le résidu est repris 3 fois par le méthanol. On élimine le solvant par évaporation sous pression réduite.
   Le résidu est dissous dans le minimum de tétrahydrofuranne et purifié par chromatographie sur plaque de silice, l'éluant étant un mélange dichlorométhane/méthanol 9:1. La bande fluorescente sous irradiation UV à 320 nm est recueillie.
   On obtient l'ester méthylique de l'acide 6-[3-(1-adamantyl) -4-méthoxyphényl]-2-naphtoïque (96mCi) marqué au tritium en position 5 du groupement adamantyl méthoxyphényl et en position 5 du groupement naphtoïque.
c) Le produit tritié précédent (96 mCi) est solubilisé dans 5ml de méthanol, 6 µl de soude 5N sont ajoutés et le mélange résultant est chauffé à reflux pendant 3 heures, refroidi, acidifié à l'aide de 2ml d'acide chlorhydrique 6N, puis extrait par 50ml d'éther éthylique.

La phase éthérée est décantée, lavée à l'eau, séchée sur sulfate de magnésium anhydre, et les solvants évaporés sous pression réduite. Le résidu obtenu est dissous dans 2 ml d'éther et purifié par chromatographie liquide à haute performance (éluant : éther éthylique 100%). On obtient 83 mCi de produit, soit un rendement de saponification de 86%.

Le produit final est dissous dans 100ml de méthanol. Sa pureté est vérifiée par chromatographie sur couche mince (Silice; éluant comme ci-dessus). Une seule tache est apparente sous irradiation UV (254 et 366 nm) et lors du comptage bêta.

Par HPLC (phase inverse, colonne ZORBAX ODS, éluant : acétonitrile/tétrahydrofuranne/eau/acide trifluoracétique 43:36:21:0,2), un seul pic est détecté par UV (320 nm) et par détection bêta (scintillation liquide en continu).

L'activité spécifique déterminée par spectrophotométrie UV, puis comptage bêta, est de 33,0 Ci/mmole.

L'activité totale obtenue, mesurée par comptage au scintillateur liquide, est de 83 mCi.

### Caractéristiques de la fixation du composé de l'exemple 1 sur les récepteurs nucléaires RARs

Des cultures de cellules F9 de carcinome embryonnaire murin (habituellement 4 boîtes de 90 mm contenant au total 4-7x10⁷ cellules par condition d'incubation) sont lavées une fois avec du PBS (tampon phosphate salin) et incubées pendant 3 heures en milieu sans sérum supplémenté avec le rétinoïde marqué en absence ou en présence de rétinoïde froid homologue (pour vérifier la spécificité). Les cellules sont alors détachées avec un mélange trypsine-EDTA et numérées avec un hémocytomètre de Burker. Le nucléosol contenant les RARs est extrait à partir des noyaux purifiés selon la méthode décrite par Daly et Redfern (1987), (c'est-à-dire après traitement par l'ADNase et le NaCl 0,6M) et analysé par chromatographie d'exclusion de haute performance (HPSEC). 50µl d'extrait marqué sont injectés sur une colonne GF250 (Dupont de Nemours) de 9x250 mm. L'élution est réalisée dans un tampon 0,3M KH₂PO₄ pH 7,8 à un débit de 1ml/min. Le contenu en protéine est suivi par mesure de densité optique à 280nm. 28 fractions de 0.3 ml chacune sont collectées et comptées dans du scintillant Picofluor (Packard). Pour chaque concentration de rétinoïde le nombre de molécules liées est déterminé à partir de la surface du pic de radioactivité et la concentration de demi-saturation (C50) calculée. La calibration de poids moléculaire de la colonne est effectuée à l'aide d'albumine humaine (67kDa), d'ovalbumine (45kDa) et de myoglobine (16,8kDa). Les courbes dose-réponse et les courbes de compétition sont analysées de manière informatique par régression non-linéaire en utilisant les diverses formes de l'équation de Clark.

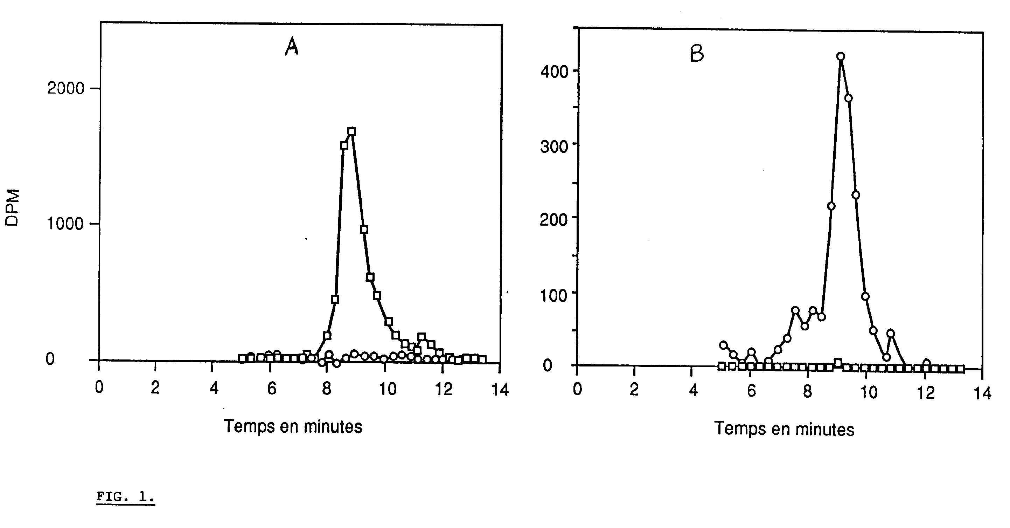

La figure 1 (A et B) représente le profil d'HPSEC dans la détection des récepteurs nucléaires de l'acide rétinoïque (RARs) par le composé de l'exemple 1 dans une fraction d'approximativement 45kDa dans le nucléosol des cellules F₉, en comparaison avec l'acide rétinoïque tritié.

Les cellules F₉ ont été incubées avec 20nM d'acide rétinoïque tritié (activité spécifique 52,5 Ci/mmole) (1A) en absence (□ - □ ) ou en présence (○ - ○) de 100 fois plus d'acide rétinoïque froid, ou avec 20nM du composé de l'exemple 1 tritié (1B) en absence (□ - □) ou présence (○ - ○) de 1000 fois plus de composé I froid. La quantité de ligand lié aux RARs est exprimée en dpm (désintégrations par minute).

Quand la méthode décrite ci-dessus est utilisée et que les cellules F9 sont incubées avec le composé de l'exemple 1 à une concentration de 20nM, un pic unique de radioactivité est observé dans l'extrait nucléaire (Figure 1B). Le poids moléculaire correspondant à la fraction moyenne du pic est de 45kDa approximativement, un chiffre proche du poids moléculaire des RARs humains, ainsi qu'il est déduit de la séquence de leurs ADNc (Petkovich et al., 1987; Brand et al., 1988), et compatible avec le coefficient de sédimentation 4S des récepteurs murins (Daly & Redfern, 1987). La fixation est spécifique puisqu'elle est abolie quand les cellules sont coincubées avec un excès de 1000 fois du composé I froid (Figure 1B).

D'autre part, par transfection de lignées cellulaires ne contenant pas de récepteurs des rétinoïdes avec des vecteurs d'expression codant isolément pour chaque récepteur des rétinoïdes, il est possible d'isoler des extraits cellulaires contenant chaque récepteur et d'effectuer un test de liaison selon le protocole décrit ci-dessus. On peut ainsi déterminer l'affinité du composé de l'exemple 1 pour chaque récepteur des rétinoïdes.

### Comparaison avec la fixation de l'acide rétinoïque tritié sur les récepteurs nucléaires RARs

Sur la figure 1A, on peut noter que le pic radioactif élue à la même position qu'avec le composé de l'exemple 1. D'autre part, cette fixation est aussi inhibée par un excès de 100 fois d'acide rétinoïque froid.

Si on normalise les résultats d'HPSEC en fonction du nombre de cellules, c'est-à-dire en les exprimant par molécules de ligand fixés aux RARs par cellule, le composé de l'exemple 1 et l'acide rétinoïque donnent un pic de même surface correspondant à approximativement 4.000 molécules par cellule.

### Caractéristiques de la fixation du composé de l'exemple 1 sur la protéine de liaison CRABP

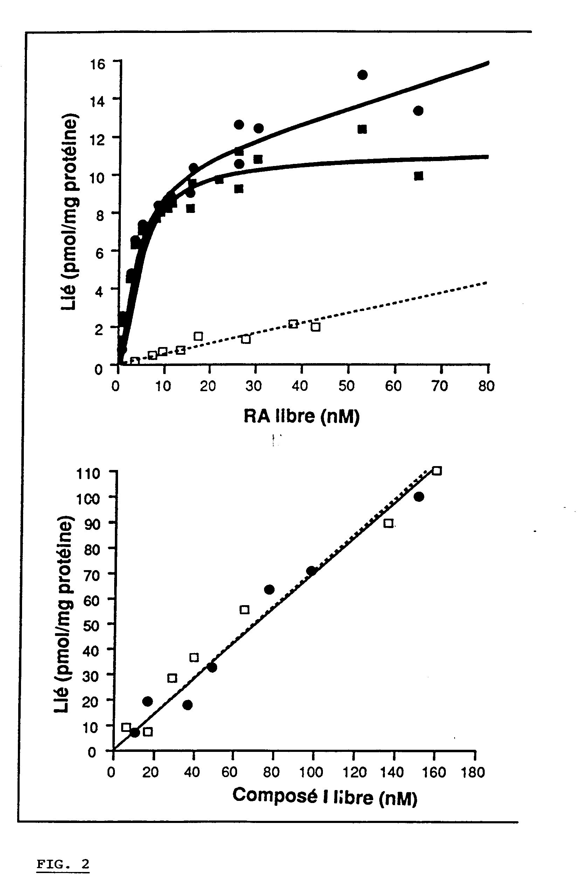

Les résultats concernant la fixation du composé I et de l'acide rétinoïque sur la CRABP sont représentés à la Figure 2. On étudie la fixation directe de concentrations croissantes du composé de l'exemple 1 (fig.2, courbe du bas) et d'acide rétinoïque (fig.2, courbe du haut) à du cytosol de testicule de rat contenant la CRABP en absence (● - ●) ou en présence de 1000 nM de ligand froid homologue (□ - □). La différence (■ - ■) représente la liaison spécifique.

La fixation du composé revendiqué sur la CRABP a été comparée à celle de l'acide rétinoïque. La fixation totale (non spécifique + spécifique) (● - ●) est obtenue par incubation de concentrations croissantes (jusqu'à un maximum de 125 nM) du composé revendiqué avec une quantité constante (0,3 ml) d'un homogénat de testicules de rat (organe riche en CRABP). La fixation non spécifique du composé tritié est déterminée en parallèle, par la mesure de la fixation avec des concentrations croissantes du ligand radioactif en présence d'un large excès (1 µM) de ligand froid homologue (□ - □). Un temps d'incubation de deux heures est nécessaire à l'établissement de conditions d'équilibre.

Une fois l'équilibre atteint, le complexe CRABP-ligand tritié est séparé du ligand non lié par filtration sur colonne de gel d'exclusion Sephadex G25 (colonnes BIO-Rad econo, 0,5 x 20 cm) : le complexe est élué (pic d'élution à 2,5 ml), tandis que le ligand non lié est retenu en totalité sur la colonne. Il en résulte une séparation totale entre produits lié et non lié. L'élution est faite directement dans des tubes à scintillation et la radioactivité est mesurée par comptage.

Sur la figure 2 en bas, on peut remarquer que le composé revendiqué ne présente aucune fixation spécifique sur la CRABP, les courbes de fixation spécifique et non spécifique étant totalement superposables.

### Comparaison avec l'acide rétinoïque tritié

Dans les mêmes conditions, on peut étudier la fixation de l'acide rétinoïque, sur la CRABP (figure 2 en haut). Sur la figure 2, on a représenté en abscisses les concentrations d'acide rétinoïque libre, et en ordonnées, les concentrations à l'équilibre du complexe ligand-protéine de liaison.

La différence entre la courbe de fixation totale (● - ●) et de fixation non spécifique (□ - □) donne la courbe de fixation spécifique (■ - ■). L'analyse de cette courbe permet d'obtenir la constante de dissociation à l'équilibre (Kd) qui est la concentration de produit nécessaire pour saturer 50% de la CRABP. Cette constante est une mesure de l'affinité du ligand pour sa protéine de liaison, le Kd étant inversement proportionnel à l'affinité (plus le Kd est faible, plus l'affinité est grande). Cette analyse donne pour l'acide rétinoïque une valeur de Kd de 2 nM. La fixation spécifique est saturable et réversible. La fixation non spécifique est linéaire et non saturable dans la gamme de concentrations utilisées, et représente moins de 20% de la fixation totale.

## Revendications

1. Acide 6-[3-(1-adamantyl)-4-méthoxyphényl]-2-naphtoïque marqué au tritium.

2. Acide 6-[3-(1-adamantyl-4- méthoxyphényl]-2-naphtoïque selon la revendication 1 caractérisé par le fait qu'il est marqué en position 5 du groupement adamantyl méthoxyphényl et en position 5 de la partie naphtoïque.

3. Acide 6-[3-(1-adamantyl)-4-méthoxyphényl]-2-naphtoïque tritié, tel que défini dans les revendications précédentes, caractérisé par le fait qu'il possède une activité spécifique d'au moins 370 GBq/mmole, en particulier d'au moins 1110 GBq/mmole.

4. Procédé de préparation d'un produit tritié tel que défini dans l'une quelconque des revendications précédentes, caractérisé par le fait que l'on prépare un alkyl ester multihalogéné d'acide 6-[3-(1-adamantyl)- 4-méthoxyphényl]-2-naphtoïque et qu'on le soumet à l'action d'un agent réducteur tritié puis à l'action d'un agent de saponification.

5. Procédé selon la revendication 4, caractérisé par le fait que l'agent réducteur est le tritium.

6. Procédé selon selon l'une quelconque des revendications 4 et 5, caractérisé par le fait que l'ester multihalogéné est obtenu par l'action d'un agent d'halogénation sur un alkyl ester de l'acide 6-[3-(1-adamantyl)- 4-méthoxyphényl]-2-naphtoïque.

7. Procédé selon l'une quelconque des revendications 4 à 6, caractérisé par le fait que l'agent d'halogénation est un agent de bromation.

8. Procédé selon l'une quelconque des revendications 4 à 6, caractérisé par le fait que l'alkyl ester utilisé est l'ester méthylique.

9. Produit intermédiaire pour la mise en oeuvre du procédé selon l'une quelconque des revendications 4 à 8, caractérisé par le fait qu'il s'agit de l'acide 6-[3-(1-adamantyl)-4 méthoxy-5-halogénophényl]-5 halogéno-2-naphtoïque.

10. Produit intermédiaire de la revendication 9, caractérisé par le fait qu'il est dibromé.

11. Utilisation de l'acide 6-[3-(1-adamantyl)-4-méthoxy-5-halogénophényl]-5-halogéno-2-naphtoïque marqué au tritium, tel que défini dans l'une quelconque des revendications 1 à 3, comme marqueur radioactif.

12. Utilisation de l'acide 6-[3-(1-adamantyl)-4-méthoxyphényl]- 2-naphtoïque tritié :
- comme marqueur radioactif pour repérer, doser ou marquer un (ou des) récepteurs nucléaires de l'acide rétinoïque endogènes ou obtenus par des méthodes de transfection, en particulier dans les extraits contaminés par de la CRABP ;
- comme marqueur radioactif pour mesurer l'affinité de substances telles que des rétinoïdes pour les RARs, selon une méthode de compétition ;
- comme marqueur radioactif pour la caractérisation d'anticorps dirigés contre ce composé, et utilisation conjointe de ce composé et desdits anticorps pour réaliser un radioimmunodosage ;
- comme marqueur radioactif pour étudier la distribution in vivo ainsi que cellulaire et subcellulaire de ce composé, sans interférence avec la CRABP ;
- comme marqueur radioactif pour étudier le métabolisme de ce composé in vivo et dans les cellules et extraits cellulaires.

## Claims

1. Tritium-labelled 6-[3-(1-adamantyl)-4-methoxyphenyl]-2-naphthoic acid.

2. 6-[3-(1-Adamantyl-4-methoxyphenyl]-2-naphthoic acid according to Claim 1, characterised in that it is labelled in position 5 of the adamantylmethoxyphenyl group and in position 5 of the naphthoic part.

3. Tritiated 6-[3-(1-adamantyl)-4-methoxyphenyl]-2-naphthoic acid as defined in the preceding claims, characterised in that it has a specific activity of at least 370 GBq/mmol, in particular of at least 1110 GBq/mmol.

4. Process for the preparation of a tritiated product as defined in any one of the preceding claims, characterised in that a polyhalogenated alkyl ester of 6-[3-(1-adamantyl)-4-methoxyphenyl]-2-naphthoic acid is prepared and in that it is subjected to the action of a tritiated reducing agent and then to the action of a saponifying agent.

5. Process according to Claim 4, characterised in that the reducing agent is tritium.

6. Process according to either of Claims 4 and 5, characterised in that the polyhalogenated ester is obtained by the action of a halogenating agent on an alkyl ester of 6-[3-(1-adamantyl)-4-methoxyphenyl]-2-naphthoic acid.

7. Process according to any one of Claims 4 to 6, characterised in that the halogenating agent is a brominating agent.

8. Process according to any one of Claims 4 to 6, characterised in that the alkyl ester employed is the methyl ester.

9. Intermediate product for making use of the process according to any one of Claims 4 to 8, characterised in that it is a 6-[3-(1-adamantyl)-4-methoxy-5-halophenyl]-5-halo-2-naphthoic acid.

10. Intermediate product of Claim 9, characterised in that it is dibrominated.

11. Use of a tritium-labelled 6-[3-(1-adamantyl)-4-methoxy-5-halophenyl)-5-halo-2-naphthoic acid as defined in any one of Claims 1 to 3, as radioactive marker.

12. Use of tritiated 6-[3-(1-adamantyl)-4-methoxyphenyl]-2-naphthoic acid:
- as radioactive marker for locating, determining or labelling one (or more) nuclear receptors for retinoic acid which are endogenic or are obtained by transfection methods, in particular in extracts contaminated with CRABP;
- as radioactive marker for measuring the affinity of substances such as retinoids for the RARs, by a competition method;
- as radioactive marker for the characterisation of antibodies directed against this compound, and combined use of this compound and of the said antibodies for carrying out a radioimmunoassay;
- as radioactive marker for studying the in-vivo and cellular and subcellular distribution of this compound, without interference with the CRABP;
- as radioactive marker for studying the metabolism of this compound in vivo and in cells and cell extracts.

## Patentansprüche

1. 6-[3-(1-Adamantyl)-4-methoxyphenyl]-2-naphtoesäure markiert mit Tritium.

2. 6-[3-(1-Adamantyl)-4-methoxyphenyl]-2-naphtoesäure nach Anspruch 1, dadurch gekennzeichnet, daß sie in Position 5 der Adamantylmethoxyphenylgruppe und in Position 5 des Naphtoeteils markiert ist.

3. 6-[3-(1-Adamantyl)-4-methoxyphenyl]-2-naphtoesäure tritiiert wie in den vorhergehenden Ansprüchen definiert, dadurch gekennzeichnet, daß sie mindestens eine spezifische Aktivität von 370 GBq/mmol, insbesondere mindestens 1110 GBq/mmol, aufweist.

4. Verfahren zur Herstellung eines tritiierten Produktes wie in einem der vorhergehenden Ansprüche definiert, dadurch gekennzeichnet, daß man einen mehrfach halogenierten Alkylester von 6-[3-(1-Adamantyl)-4-methoxy-phenyl] -2-naphtoesäure herstellt, mit einem tritiierten Reduktionsmittel behandelt, anschließend mit einem Verseifungsmittel behandelt.

5. Verfahren nach Anspruch 4, dadurch gekennzeichnet, daß das Reduktionsmittel Tritium ist.

6. Verfahren nach einem der Ansprüche 4 und 5, dadurch gekennzeichnet, daß man den mehrfach halogenierten Ester durch Einwirken eines Halogenierungsmittels auf einen Alkylester von 6[-3-(1-Adamantyl)-4-methoxy-phenyl]-2-naphtoesäure erhält.

7. Verfahren nach einem der Ansprüche 4 bis 6, dadurch gekennzeichnet, daß das Halogenierungsmittel ein Bromierungsmittel ist.

8. Verfahren nach einem der Ansprüche 4 bis 6, dadurch gekennzeichnet, daß der verwendete Alkylester der Methylester ist.

9. Zwischenprodukt zur Verwendung in einem der Ansprüche 4 bis 8, dadurch gekennzeichnet, daß es sich dabei um 6-[3-(1-Adamantyl)-4-methoxy-5-halogenphenyl]-5-halogen-2-naphtoesäure handelt.

10. Zwischenprodukt nach Anspruch 9, dadurch gekennzeichnet, daß es dibromiert ist.

11. Verwendung von 6 [-3-(1-Adamantyl)-4-methoxyphenyl]-2-naphtoesäure markiert mit Tritium, wie in einem Ansprüche 1 bis 3 definiert, als radioaktiven Marker.

12. Verwendung von tritiiertem 6[-3-(1-Adamantyl)-4-methoxyphenyl]-2-naphtoesäure als:
- radioaktive Markierung zur Lokalisierung, Bestimmung oder Markierung eines (oder der) endogenen Nuklearrezeptors(en) der Retinoesäure oder erhalten durch Transfektionsverfahren, insbesondere in durch CRABP kontaminierten Extrakten;
- radioaktive Markierung zur Messung der Affinität nach einem Kompetitionsverfahren von Substanzen wie der Retinoide für die RARs;
- radioaktive Markierung zur Charaktisierung der gegen die Verbindung gerichteten Antikörper und zur Verwendung gemeinsam mit der Verbindung und den Antikörpern zur Durchführung einer Radioimmunbestimmung;
- radioaktive Markierung zur Untersuchung der zellulären und subzellulären Verteilung der Verbindung in vivo ohne Interferenz mit CRABP;
- radioaktive Markierung zur Untersuchung des Metabolismus der Verbindung in vivo und in den Zellen und in zellulären Extrakten.
